## Europäisches Patentamt

## European Patent Office

## Office européen des brevets

(11) Publication number: **0 089 198**
**A2**

---

(12) # EUROPEAN PATENT APPLICATION

(21) Application number: **83301359.2**

(22) Date of filing: **11.03.83**

(51) Int. Cl.³: **A 61 K 31/60**
**A 61 K 31/615**
**//(A61K31/60, 31/195)**

---

(30) Priority: **11.03.82 US 357074**

(43) Date of publication of application:
**21.09.83 Bulletin 83/38**

(84) Designated Contracting States:
**BE CH DE FR GB IT LI LU NL**

(71) Applicant: **DYNATECH LABORATORIES,
INCORPORATED**
**900 Slater Lane**
**Alexandria Virginia 22314(US)**

(72) Inventor: **Bender, Charles E.**

**deceased(US)**

(74) Representative: **Hose, Cyril Gustav Bidwell et al,
Baron & Warren 18 South End Kensington
London W8 5BU(GB)**

---

(54) **Nontoxic aspirin-containing composition.**

(57) An analgesic aspirin-containing composition comprising acetyl-salicylic acid and glycine, said glycine being present in an amount equal to at least substantially 33.3% of the weight of the acetylsalicylic acid.

EP 0 089 198 A2

Croydon Printing Company Ltd.

"NONTOXIC ASPIRIN-CONTAINING COMPOSITION"

This invention relates to a pharmaceutical composition comprising analgesic acetyl-salicylic acid (aspirin).

Pure acetyl-salicylic acid (aspirin) has been the most commonly used medication since its introduction many years ago. It has been regarded by the public as the thing nearest to a completely safe drug available on the market.

While aspirin taken occassionally in limited dosages is relatively safe, it has certain disadvantages. It has a bitter taste, so that it is generally taken in the form of pills rather than in solution or as a powder. Moreover, when used regularly, it has undesirable side effects which limit its utility. When massive dosages are needed, as in the case of chronic arthritis, these side effects - generally known as salicylate poisoning - often limit the dosages used to a level at which they are no longer effective. In many cases, particularly when a patient has an underlying mild-to-moderate chronic atrophic gastritis, aspirin aggravates the condition to cause the gastritis to become haemorrhagic. This effect also seems to be due to the conversion of the aspirin to salicylic acid salts, and is related to the appearance of salicylates in the blood stream.

It has been proposed (U.S. Patent No. 2,933,821) to admix aspirin with lysine in order to overcome its bitter taste, and produce an aspirin composition without the characteristic bitter taste of aspirin. However, this admixture has no effect on the toxic side effects of aspirin - it still degrades the salicylic acid _in vivo_,

and the salicylates which appear in the bloodstream have the same toxic effects as when unblended aspirin is used.

It has now been found that acetyl-salicylic acid, when admixed with 33.3% to 222% of its weight of glycine, not only loses its bitter taste, but is also prevented from breaking down into salicylates in the bloodstream, so that the common toxic side effects of aspirin are overcome.

This invention is based on the discovery that glycine suppresses the in vivo breakdown of acetyl-salicylic acid to a salicylic acid and its salts if glycine is mixed with acetyl-salicylic acid in an amount equal to at least 33.3% of the weight of the acetyl-salicylic acid that is present in the composition and preferably in an amount equal to substantially 55.6% of the weight of the acetyl-salicylic acid. For an analgesic aspirin composition containing 90% by weight of acetyl-salicylic acid and 10% by weight of starch, the amount of glycine used is equal to at least 30% of the weight of the aspirin composition and is preferably present in an amount equal to 50% of the weight of the aspirin composition. We may use more glycine without impairing the therapeutic effect of the aspirin, but for cost reasons, we prefer not to use glycine in an amount in excess of substantially 200% of the weight of the 90% pure aspirin composition mentioned above. Mixing glycine with the acetyl-salicylic acid in an amount equal to at least 33.3% of the weight of the acetyl-salicylic acid which is present in the composition is sufficient substantially to suppress the _in vivo_ breakdown of the acetyl-salicylic acid into salicylic acid and its salts without impairing the therapeutic effect of acetyl-salicylic acid.

In addition to the inert materials normally present in the aspirin tablets, we may add small amounts of taste-improving additives, such as, mannitol, sugars, and various flavouring agents. The pH of the tablet when dissolved can be adjusted to be near to the neutral point to produce an aspirin tablet which can be swallowed whole but which, in addition, can be sucked, chewed, or allowed to dissolve sublingually.

Extensive clinical testing of the medicament over a period of time indicates that the medicament of this invention is as effective as ordinary aspirin in relieving pain. A group of fifty patients, ranging in age from 5 to 76, was treated with 5 mg aspirin tablets having the following composition, based on the total weight of the formulation: aspirin composition (containing 90% by weight of acetyl-salicylic acid and 10% by weight of starch) --
64.6%;
glycine (amino acetic acid) —— 31.3%;
sodium saccharin —— 0.4%;
sodium cyclomate —— 3.6% and
lemon flavour (FLAVO-LOCK) —— 0.1%.

In general, young children preferred a sweeter-tasting product; pain was relieved in most cases, but not in all, as in a similar group of control patients tested with ordinary aspirin. Significantly, of the group treated with the present medicament there were five patients with records of gastritis, or stomach or intestinal ulcers, all of whom suffered upset stomachs or bleeding under aspirin therapy. None of them reported any such symptoms when treated with the above composition.

In place of sodium saccharin, a like amount of sodium cyclomate may be used in the above composition, making the total amount of sodium cyclomate present equal to 4.0% of the total weight of the composition. Alternatively, the sodium cyclomate may be replaced by a like amount of sodium saccharin in the above composition, making the total amount of sodium saccharin present equal to 4.0% of the total weight of the composition.

The compositions of the present invention are non-toxic admixtures of acetyl-salicylic acid and glycine which are free from water and are prepared for ingestion as an admixture. When prepared in the form of tablets they constitute unit doses of an orally ingestible admixture. In the compositions of the present invention the acetyl-salicylic acid and the glycine are present as such and no reaction has taken place between these two ingredients. Moreover the glycine content of these admixtures should be sufficient to suppress the _in vivo_ breakdown of the acetyl salicylic acid content without substantial impairment of the therapeutic effect of the acetyl-salicylic acid. In the absence of the glycine acetyl-salicylic acid breaks down and forms salicylic-acid and its salts in the human stomach. However the glycine present in the compositions renders the compositions insoluble in the acidic environment provided by the gastric juices present in the human stomach and in this way suppress the _in vivo_ breakdown of the acetyl-salicylic acid.

# C L A I M S

1. An orally administrable aspirin composition comprising anhydrous acetyl-salicylic acid characterised in that, anhydrous glycine is admixed with said acid in an amount sufficient to suppress the in vivo breakdown of said acetyl-salicylic acid to salicylic acid and its salts without substantially impairing the therapeutic effect of the acetyl-salicylic acid.

2. An orally administrable aspirin composition according to claim 1, characterised in that it comprises 100 parts by weight of anhydrous acetyl-salicylic acid and at least 33 parts by weight of anhydrous glycine admixed with said acid, said amount of said glycine being sufficient to suppress the in vivo breakdown of said acetyl-salivylic acid to salicylic acid and its salts without substantially impairing the therapeutic effect of the acetyl-salicylic acid.

3. An orally administrable aspirin composition according to claim 2, further characterised in that the amount of glycine present is equal to substantially 55.6% of the weight of said acetyl-salicylic acid.

4. A composition according to any of the preceding claims in the form of a tablet.

5. An orally administrable aspirin composition comprising anhydrous acetyl-salicylic acid characterised in that it comprises an anhydrous mixture of acetyl-salicylic acid and glycine, the glycine being unreacted with the acetyl-salicylic acid prior to ingestion, said anhydrous glycine being present in an amount sufficient to suppress the in vivo breakdown of said acetyl-salicylic acid to salicylic acid and its salts in a human stomach after ingestion by a human being without substantially
impairing the therapeutic effect of the acetyl-salicylic
acid.

6. A composition according to claim 5 further
characterised in that it additionally includes starch.

7. A composition according to either of claims 5 or 6,
in the form of a tablet.